Europäisches Patentamt

European Patent Office

Office européen des brevets

(19)

(11) Publication number: **0 101 945**
**B1**

(12) **EUROPEAN PATENT SPECIFICATION**

(45) Date of publication of patent specification: **27.08.86**

(21) Application number: **83107440.6**

(22) Date of filing: **28.07.83**

(51) Int. Cl.⁴: **C 12 Q 1/28,** C 12 Q 1/26,
C 12 Q 1/54, C 12 Q 1/00,
C 12 Q 1/60, G 01 N 33/52

(54) **Multilayer analytical element.**

(30) Priority: **28.07.82 JP 131750/82**

(43) Date of publication of application:
**07.03.84 Bulletin 84/10**

(45) Publication of the grant of the patent:
**27.08.86 Bulletin 86/35**

(84) Designated Contracting States:
**DE GB**

(56) References cited:
**EP-A-0 033 707**
**US-A-4 098 574**
**US-A-4 255 384**

(73) Proprietor: **FUJI PHOTO FILM CO., LTD.**
**210 Nakanuma Minamiashigara-shi**
**Kanagawa-ken, 250-01 (JP)**

(72) Inventor: **Arai, Fuminori**
**Fuji Photo Film Co., Ltd. 3-11-46 Senzui**
**Asaka-shi Saitama (JP)**
Inventor: **Kitajima, Masao**
**Fuji Photo Film Co., Ltd. 3-11-46, Senzui**
**Asaka-shi Saitama (JP)**

(74) Representative: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

# 0 101 945

## Description

Background of the invention
Field of the invention

This invention relates to a multilayer analytical element. More particularly, this invention relates to an improvement of a multilayer analytical element containing a color indicator system and peroxidase for detection of hydrogen peroxide which is appropriately employable in quantitative analysis of hydrogen peroxide in a sample liquid, or in quantitative analysis of a specific component through quantitative determination of the amount of hydrogen peroxide produced upon contact of the specific component with an oxidase enzyme system.

Description of prior arts

A quantitative analysis of a specific component (referred to herein as "analyte") based on quantitative determination of the amount of $H_2O_2$ produced by an oxidation reaction between the analyte or a reaction product of the analyte and an oxidase enzyme, by means of an appropriate determination procedure has recently become more important. The reason is that the quantitative determination of $H_2O_2$ can be made accurately and reliably, for instance, by photometric determination of a colored product formed by the action of $H_2O_2$ in the presence of peroxidase or determination of $H_2O_2$ utilizing an electrode reaction.

As for the photometric determination method based on the above-mentioned principle, there is known a method using a reagent system proposed by P. Trinder (See Ann. Clin. Biochem., 6, 24—27 (1969)). This method involves: producing $H_2O_2$ by a reaction between an analyte and an oxidase enzyme; causing an oxidative coupling reaction between 4-aminoantipyrine (or an analogue thereof) and a phenol (or a naphthol) in the presence of $H_2O_2$ and peroxidase to prdouce a colored product; and quantitatively determining the so produced colored product. This reaction system is advantageous because the same detection system is employable regardless of varying the oxidase enzyme. Accordingly, this reaction system has been studied for application in detection of various analytes. Examples of analytes particularly important in the art of clinical chemical tests include glucose oxidase, cholesterol oxidase, uricase, glycerol oxidase, and phosphoglucose oxidase.

An analytical element employing said oxidase enzyme and a detection system for the produced $H_2O_2$ in the form of an integral multilayer analytical element of a strip (such as a filter power strip) impregnated with these reagents is widely employed for clinical tests. This analytical element comprises a composition containing reagents directly participating in the detection of an analyte which is impregnated in a filter paper strip or the like or coated over a filmy support.

The integral multilayer analytical element can be in a variety of constitutions, and different functional layers can be incorporated into the element under lamination as need arises. Examples of known functional layers include; a spreading layer for uniform spreading of a sample liquid (US Patent 3,992,158, GB 1,440,464, and US Patent 4,292,272); a light-shielding layer providing a light-scattering white surface for reflection measurement, as well as serving for photometrically separating the colored product produced upon the reaction from colored components contained in the upper layer (US Patent 3,992,158, GB 1,440,464, and US Patents 4,042,335, 4,255,384, and 4,292,274, etc.); a barrier layer for selective diffusion of component, product, etc. (US Patent (Re) 30261) or a liquid-blocking air barrier layer for the same purpose (Japanese Patent Provisional Publications No. 58(1983)—77660 and No. 58(1983)—776615); a mordant detection layer for efficient detection of the colored prdocut (US Patents 4,042,335 and 4,144,306); a migration inhibiting layer for preventing transfer of the colored product into other layers (US Patent 4,166,093); and an adhesive layer for improvement of adhesion between the incorporated layers (US Patent 4,292,272, etc.).

It is known that blood samples such as samples of whole blood, blood plasma and blood serum, contain sodium fluoride (NaF) as a preservative for preserving the blood samples for a long period such as approx. 10 days. In these blood samples, NaF is generally contained in an amount of approximately 2—10 mg. per 1 ml. of the blood sample. Thus, NaF is effective in the preservation of blood sample, but has a drawback that it likely interferes with the detection of analyte contained in the blood sample. For instance, in a quantitative determination of glucose in a blood serum containing 2.0 mg. of NaF per 1 ml. of serum, by the use of a multi-layer analytical element for glucose analysis disclosed in Example 3 of US Patent 3,992,158, the glucose content of 200 mg. per 1 dl. of the serum is analyzed to give a value less than the correct value by a value in the range of 20—30 mg./dl.

For avoiding such interference on the resulting value caused by the presence of NaF, US Patent 4,098,574 proposes an integral multilayer analytical element for analysis of glucose in which an organic acid such as 3,3-dimethylglutaric acid, succinic acid or malic acid is incorporated into a reagent layer containing, as principal components, glucose oxidase, peroxidase, 4-aminoantipyrine and 1,7-dihydroxynaphthalene for maintaining the reagent layer at pH 5.0—5.6 under analytical procedural conditions (its working examples are given in Examples 1, 2, 6 and 11).

Accordingly, the present inventors made studies on the analytical element described in the working examples of the above-mentioned US Patent 4,098,574, as well as an exemplary multilayer analytical element for glucose analysis comprising a fabric spreading layer (US Patent 4,292,272, and Japanese Patent Provisional Publication No. 55(1980)—164356) or comprising both a fabric spreading layer and

2

0 101 945

glucose oxidase contained in an adhesive layer or a light-shielding layer (GB 2 104 215A, and Japanese Patent Provisional Publication No. 57(1982)—208977) using the pH-adjusted reagent layer proposed in the above-mentioned publication. As a result of the studies, it was observed that these analytical elements suffer little interference, that is, little decrease of a measured value, in the determination of glucose in the case where the blood sample contains the NaF preservative in an amount of not more than approx. 4 mg./ml. However, it was further observed that the interference by NaF is prominent in the case where the blood sample contains NaF in an amount exceeding approx. 5 mg./ml. Furthermore, it was observed that these multilayer analytical elements give unreliable measured values in the case where the blood sample contains no or less than approx. 2 mg./ml. of NaF. In the course of these studies, the present inventors further discovered that the interference, i.e. decrease of analytically measured values, occurs not only in the multilayer analytical element for glucose analysis containing glucose oxidase and peroxidase, but also in all of multilayer analytical elements containing peroxidase.

Summary of the invention

A primary object of the present invention is to provide an improved multilayer analytical element containing a color-forming reagent composition including peroxidase for detection of $H_2O_2$ in a reagent layer, which is substantially free from the interference, i.e. decrease of measured values, caused by the presence of NaF, whereby enabling peroxidase to show high activity.

Another object of the invention is to provide an improved multilayer analytical element containing a color-forming reagent composition including oxidase and peroxidase for detection of $H_2O_2$ in a reagent layer, which is substantially free from the interference, i.e. decrease of measured values, caused by the presence of NaF, whereby enabling oxidase and peroxidase to show high activities.

A further object of the invention is to provide an improved multilayer analytical element containing a color-forming reagent composition including oxidase and peroxidase for detection of $H_2O_2$ in a reagent layer, which is substantially free from the interference, i.e. decrease of measured values, caused by the presence of NaF, whereby enabling oxidase and peroxidase to show high activities and enlarging the measurable range.

A still further object of the invention is to provide specifically an improved multilayer analytical element containing a color-forming reagent composition including glucose oxidase and peroxidase for detection of $H_2O_2$ in a reagent layer, which is substantially free from the interference, i.e. decrease of measured values, caused by the presence of NaF, whereby enabling oxidase and peroxidase to show high activities and accordingly enlarging the measurable range.

The present invention provides

(1) a multilayer analytical element comprising a water-impermeable, light-transmissive support, a reagent layer which comprises a hydrophilic binder polymer and a color-forming indicator composition for detection of hydrogen peroxide, this reagent layer containing at least peroxidase, and a porous spreading layer, which are superposed in that order, in which at least one layer other than said support contains 0.1 meq./m² to 1 eq./m² of a compound comprising a cation capable of forming in combination with F⁻ ion a sparingly water-soluble salt having solubility of not more than 0.2 g. in 100 g. of water at 25°C.

Examples of the preferred embodiments of the present invention include:

(2) The multilayer analytical element as described in the above (1), in which said cation is at least one cation selected from the group consisting of $Mg^{2+}$, $Ca^{2+}$, $Sr^{2+}$, $Ba^{2+}$, $Pb^{2+}$, $Fe^{2+}$, $Cu^{2+}$, $Bi^{3+}$, $Fe^{3+}$, $V^{3+}$, $Na_3Al^{6+}$, $K_2Si^{6+}$, $CaSi^{6+}$, and $BaSi^{6+}$;

(3) The multilayer analytical element as described in the above (1) or (2), in which said reagent layer contains peroxidase and 4-aminoantipyrine or a derivative thereof;

(4) The multilayer analytical element as described in any of the above (1) through (3), in which oxidase is contained in said reagent layer or any other layer;

(5) The multilayer analytical element as described in the above (4), in which said oxidase is selected from the group consisting of glycollate oxidase, malate oxidase, glucose oxidase, hexose oxidase, cholesterol oxidase, L-gulonolactone oxidase, galactose oxidase, xanthine oxidase, pyruvate oxidase, uricase, ascorbate oxidase, lactate oxidase, glycine oxidase, and glycerol oxidase;

(6) The multilayer analytical element as described in the above (4), in which said oxidase is glucose oxidase; and

(7) The multilayer analytical element as described in the above (4), in which said oxidase is cholesterol oxidase.

Brief description of drawing

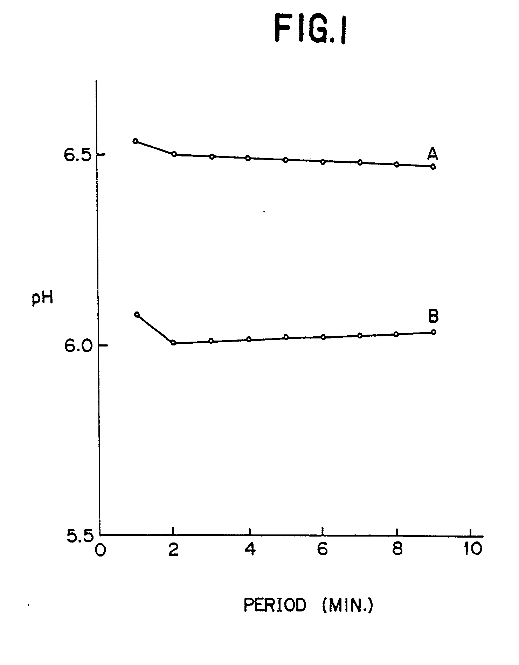

Fig. 1 graphically indicates exemplary pH value changes in the analytical element of the present invention observed with the lapse of time. The measurement of pH value was carried out by placing a pH measuring electrode in contact with an adhesive layer of the analytical element. In the Figure, A and B mean curves of pH change observed on sample liquids, fresh human plasma and distilled water, respectively.

Description of preferred embodiments

There is no specific limitation on the water-impermeable, light-transmissive support comprised in the

3

multilayer analytical element of the present invention, so far as it allows transmission of applied electromagnetic waves in the wavelength range of approx. 200 nm to approx. 900 nm, such as ultraviolet rays, near-ultraviolet rays, visible rays and near-infrared rays at a ratio of not less than approx. 40%, preferably not less than approx. 65%, permits substantially no permeation of water thereinto, and is substantially chemically inert to a binder polymer and other materials comprised in the hereinafter-described reagent layers and under-coating layer to be superposed on the support. Examples of the support include a transparent sheet or film made of a polymer such as polyethylene terephthalate, polycarbonate of bisphenol A, polymethyl methacrylate, polystyrene, cellulose esters (e.g., cellulose diacetate, cellulose triacetate, and cellulose acetate propionate) and a transparent glass plate. The thickness of the support generally ranges from approx. 50 μm to approx. 2 mm, preferably from approx. 70 μm to approx. 0.5 mm.

On a surface of the support can be provided a known under-coating layer for facilitating adhesion of a reagent layer or one of other functional layers against the support so as to form an integrated structure. For the same purpose, the surface of the support can be modified by a known chemical processing such as acid processing or alkaline processing, or a known physical processing such as corona discharge processing, glow discharge processing, ultraviolet rays irradiation processing or flame processing.

The reagent layer containing at least peroxidase which is comprised in the multilayer analytical element of the present invention is a reagent layer which comprises a hydrophilic binder polymer and a color-forming indicator composition for detection of hydrogen peroxide (referred to hereinafter as "color-forming reagent for $H_2O_2$") containing at least peroxidase, the latter being dispersed or dissolved in the matrix of the former binder polymer.

The color-forming reagent for $H_2O_2$ comprises peroxidase and an indicator (including one compound or a combination of two or more compounds) which shows a detectable change (generally, change of color density, or change of hue) in the presence of peroxidase and hydrogen peroxide.

Examples of the indicator include the following.

(1) a combination of 4-aminoantipyrine or a derivative thereof (chromogen) and phenol, naphthol, or a derivative thereof (coupler) disclosed in Ann, Clin. Biochem., 6, 24—27 (1969), US Patents 3,992,158, 3,983,005 and 4,292,272, EP 0033539 A2, EP 0033540 A2, DE 3 301 470 A1, and Japanese Patent Application No. 57(1982)—6606.

Examples of the derivative of 4-amino-antipyrine (i.e. 4 - amino - 2,3 - dimethyl - 1 - phenyl - 3 - pyrazolin - 5 - one) include 4 - amino - 2,3 - dimethyl - 1 - (3 - chlorophenyl) - 3 - pyrazolin - 5 - one, 4 - amino - 2,3 - dimethyl - 1 - (2,4,6 - trichlorophenyl) - 3 - pyrazolin - 5 - one, 4 - amino - 2 - methyl - 3 - phenyl - 1 - (2,4,6 - trichlorophenyl) - 3 - pyrazolin - 5 - one, 4 - amino - 1 - (4 - aminophenyl) - 2,3 - dimethyl - 3 - pyrazolin - 5 - one, 4 - amino - 1 - [4 - (diethylamino)phenyl] - 2,3 - dimethyl - 3 - pyrazolin - 5 - one, 4 - amino - 1 - (4 - acetamidophenyl) - 2,3 - dimethyl - 3 - pyrazolin - 5 - one, 4 - amino - 1 - (amino - 2 - ethylphenyl) - 2,3 - dimethyl - 3 - pyrazolin - 5 - one, and 4 - amino - 1 - (amino - 4 - methylphenyl) - 2,3 - dimethyl - 1 - phenyl - 3 - pyrazolin - 5 - one; in which 4 - aminoantipyrine and derivatives thereof can be employed in the form of their acid addition salts (e.g., hydrochloride).

Examples of the naphthol include 1-naphthol and 2-naphthol.

Examples of the phenol derivative include catechol, resorcinol, pyrogallol, phloroglucinol, o-, m-, or p-cresol, o-, m-, or p-methoxyphenol, o-, m-, or p-sulfophenol, and o- or p-chlorophenol.

Examples of the naphthol derivative include 1,7-dihydroxynaphthalene and 4-methoxy-1-naphthol.

Examples of the phenol derivative and naphthol derivative further include compounds described in The Theory of the Photographic Process, Third Edition, edited by C. E. K. Mees & T. H. James (Macmillan Co., New York, 1966), pp. 387—396.

Preferred combinations include a combination of 4-aminoantipyrine and 1,7-dihydroxynaphthalene and a combination of 4-amino-2,3-dimethyl-1-(2,4,6-trichlorophenyl)-3-pyrazolin-5-one and 1,7-dihydroxynaphthalene.

(2) A combination of a 5-pyrazolone derivative or 4-oxazolone derivative (chromogen) and a phenol or a naphthol (coupler) as disclosed in Japanese Patent Publication No. 55(1980)—25840.

(3) A combination of a 4-aminopyrazolone derivative such as 1-(4-sulfophenyl-2-methyl-3-phenyl-4-amino-3-pyrazolin-5-one (chromogen) and phenol, a phenol derivative or a polyvalent phenol such as catechol, resorcinol, hydroquinone, cresol, guaiacol, pyrogallol, orcinol, or gallic acid (coupler) as disclosed in Japanese Patent Provisional Publication No. 49(1974)—114987.

(4) A combination of a (p-toluenesulfonylhydrazino)benzothiazolium, quinolinium or pyridinium derivative (dye forming compound) and phenol, naphthol, a derivative thereof, an aromatic amine, or a reactive methylene-containing compound as disclosed in US Patent 4,089,747. A triarylimidazole derivative serving as such a dye precursor disclosed in said Publication is employed even singly.

(5) A combination of 4-amino-antipyrine, a 4-substituted antipyrine, an N,N-di-substituted-o- or p-phenylenediamine, a 2-hydrozonobenzothiazoline or a p-halogenophenol (hydrogen doner, chromogen) and an N,N-di-substituted aniline (coupler) as disclosed in GB 2 095 401A and Japanese Patent Provisional Publication No. 57(1982)—142562.

(6) A combination of an o- or p-aminophenol compound or an o- or p-phenylenediamine compound and a non-diffusion (diffusion-resistant) phenol compound, a nondiffusion naphthol compound, a nondiffusion acylacetamide compound or a nondiffusion pyrazolone compound as disclosed in Japanese Patent Provisional Publications No. 57(1982)—94653, No. 57(1982)—94654, No. 57(1982)—94655, and No. 56(1982)—94656.

(7) A color-forming substrate of peroxidase as disclosed in US Patent 3,983,005. If necessary, a coupler is also employed.

Among these indicators, the combination of 4-aminoantipyrine or a derivative thereof and naphthol or a derivative thereof is preferred. Particularly preferred are a combination of 4-aminoantipyrine and 1,7-dihydroxynaphthalene as well as a combination of 4-amino-2,3-dimethyl-1-(2,4,6-trichlorophenyl)-3-pyrazolin-5-one and 1,7-dihydroxynaphthalene.

Peroxidase can be a peroxidase of plant or animal origin (EC 1.11.7) as disclosed in US Patents 3,983,005 and 4,211,845, GB 2 036 963A, and others, or a peroxidase of microorganism origin (EC 1.11.7) as disclosed in Japanese Patent Provisional Publication No. 57(1982)—99192 and others. The peroxidase can be employed alone or in combination.

Examples of the peroxidase of plant origin include peroxidases extracted from horseradish, potato, fig tree sap, turnip, and Japanese radish. Examples of the peroxidase of animal origin include lacto-peroxidase extracted from milk and verdoperoxidase extracted from white corpuscle. Examples of the peroxidase of microorganism origin include peroxidase originating from microorganisms capable of producing non-specific peroxidase belonging to Genus Alternaria, Genus Cochliobolus, Genus Curvularia, or Genus Pellicularia.

Among these peroxidases, peroxidases of plant origin and non-specific peroxidases of microorganism origin are preferred.

As described hereinbefore, the reagent layer containing at least peroxidase which is comprised in the multilayer analytical element of the present invention is a reagent layer which comprises a hydrophilic binder polymer and a color-forming indicator composition dispersed or dissolved in the binder.

There is no specific limitation on a hydrophilic polymer employable as the binder polymer, so far as the polymer is capable of forming a film and is substantially inert to peroxidase and the indicator composition. Examples of the hydrophilic polymer include gelatin, acid-processed gelatin, deionized gelatin, gelatin derivatives such as acylated gelatin, poly(vinyl alcohol), poly(vinylpyrrolidone), poly(sodium vinylbenzene-sulfonate), carboxymethyl cellulose, sodium carboxymethylcellulose, hydroxyethylcellulose, methylcellulose, pulluran, pullulan derivatives, polyacrylamide, and acrylamide copolymers such as acrylamide—N-vinylpyrrolidone copolymer and acrylamide—2-hydroxyethylacrylamide copolymer. The thickness (dry basis) of the reagent layer generally ranges from approx. 5 μm to approx. 100 μm, preferably from approx. 10 μm to approx. 50 μm.

The peroxidase contained in the reagent layer generally amounts to from approx. 5,000 $U/m^2$ to approx. 100,000 $U/m^2$, preferably from approx. 10,000 $U/m^2$ to approx. 60,000 $U/m^2$.

The indicator composition is necessarily incorporated in an amount equivalent to or more than the amount stoichiometrically corresponding to a presumed maximum amount of the analyte contained in the sample liquid under analysis. The amount of the indicator composition can be determined by those skilled in the art through experimental trials.

There will be given description hereunder on the compound comprising a cation capable of forming, in combination with $F^-$ ion, a sparingly water-soluble salt having solubility of not more than 0.2 g. in 100 g. of water at 25°C. Said compound and sparingly water-soluble salt are referred to hereinafter as "sparingly soluble F salt-forming compound" and "sparingly soluble F salt", respectively.

The sparingly soluble F salt-forming compound is included in at least one layer other than the support of the multilayer analytical element of the present invention. In the case where the sparingly soluble F salt-forming compound is included in a layer comprising a hydrophilic binder polymer, said compound is preferably dispersed or dissolved in the hydrophilic binder polymer. The sparingly soluble F salt-forming compound preferably is as such soluble or dispersible in water, because the layer containing said compound in the hydrophilic binder polymer is generally prepared using an aqueous coating solution or dispersion in which the sparingly soluble F salt-forming compound is dissolved or dispersed in an aqueous solution of the hydrophilic polymer.

The cation comprised in the sparingly soluble F salt-forming compound is selected from cations substantially inert to peroxidase and oxidase, the latter being optionally incorporated into one of the layers of the multilayer analytical element, or at least not so active to extremely reduce the activity of these enzymes. Examples of the cation include divalent cations of alkaline earth metals such as $Mg^{2+}$, $Ca^{2+}$, $Sr^{2+}$, and $Ba^{2+}$; divalent or polyvalent cations of other metals such as $Pb^{2+}$, $Fe^{2+}$, $Cu^{2+}$, $Bi^{3+}$, $Fe^{3+}$, $V^{3+}$; and polyvalent cations containing two or more atoms such as $Na_3Al^{6+}$, $K_2Si^{6+}$, $CaSi^{6+}$, and $BaSi^{6+}$. The cations may be certain hydrated cations. Preferred are $Mg^{2+}$ and $Ca^{2+}$ cations, because these cations also serve for stabilizing the enzymes such as peroxidase and oxidase.

There is no specific limitation on the counterpart anion comprised in the sparingly soluble F salt-forming compound, so far as the compound formed by the combination between said anion and the above-mentioned cation is soluble or not sparingly soluble in water, and further so far as the anion is substantially inert to peroxidase and oxidase, the latter being optionally incorporated into one of the layers of the multilayer analytical element, or the anion is at least not so active to extremely reduce the activity of these enzymes.

5

Examples of the counterpart anion include lower aliphatic monocarboxylate anions such as formate ion, acetate ion, propionate ion, butyrate ion, isobutyrate ion, valerate ion, isovalerate ion, pivalate ion, and phenylacetate ion; lower aliphatic dicarboxylate anions such as oxalate ion, malonate ion, succinate ion, methylsuccinate ion, glutarate ion, α-methylglutarate ion, β-methylglutarate ion, α,α'-dimethylglutarate ion, 3,3-dimethylglutarate ion, adipate ion, and pimelate ion; aliphatic polycarboxylate anions such as camphoronate ion and propane-1,2,3-tricarboxylate ion; carbocyclic carboxylate anions such as camphorate ion, camphoate ion, benzoate ion, phthalate ion, isophthalate ion, and terephthalate ion; hydroxycarboxylate anions such as glycolate ion, lactate ion, glycerate ion, tartronate ion, malate ion, tartrate ion, citrate ion, benzilate ion, and salicylate ion; halogen anions such as chloride ion and bromide ion; borate ion; nitrate ion, phosphate ion, sulfate ion, thiosulfate ion, nitrite ion, and azide anion ($N_3^-$). Among these anions, preferred are lower aliphatic monocarboxylate anions, lower aliphatic dicarboxylate anions, hydroxycarboxylate anions, halogen anions, phosphate ion, nitrate ion, and sulfate ion. Particularly preferred are acetate ion, propionate ion, glutarate ion, α-methylglutarate ion, β-methylglutarate ion, α,α'-dimethylglutarate ion, 3,3-dimethylglutarate ion, malate ion, tartrate ion, citrate ion, chloride ion, and phosphate ion. The polyvalent counterpart anions can be employed in the form of polyvalent anions containing no proton or acidic anions containing one or more protons.

The sparingly soluble F salt-forming compound is a compound in which the above-mentioned cation and counterpart anion are combined. Said compound can comprise water or alcohol of crystallization. Preferred examples of the sparingly soluble F salt-forming compound include calcium formate, calcium acetate, calcium propionate, calcium succinate, calcium methylsuccinate, calcium glutarate, calcium α-methylglutarate, calcium β-methylglutarate, calcium α,α'-methylglutarate, calcium 3,3-dimethylglutarate, calcium benzoate, calcium malate, calcium tartrate, calcium citrate, calcium chloride, calcium dihydrogenphosphate, calcium nitrate, magnesium formate, magnesium acetate, magnesium propionate, magnesium succinate, magnesium methylsuccinate, magnesium glutarate, magnesium α-methylglutarate, magnesium β-methylglutarate, magnesium α,α'-methylglutarate, magnesium 3,3-dimethylglutarate, magnesium malate, magnesium tartrate, magnesium citrate, magnesium salicylate, magnesium chloride, magnesium magnesium nitrate, magnesium dihydrogenphosphate, magnesium sulfate, barium acetate, cupric acetate, and ferrous acetate.

The sparingly soluble F salt-forming compound is contained in the multilayer analytical element in such a manner that said compound forms a sparingly water-soluble salt with the $F^-$ ion in the analytical procedural conditions, whereby substantially removing the $F^-$ ion from an area where peroxidase catalyzes color forming reaction of the indicator. In other words, the sparingly soluble F salt-forming compound can be included in the reagent layer containing peroxidase, or in any layer (including a porous spreading layer) provided over the reagent layer containing peroxidase, that is, any layer placed on the side opposite to the support with respect to the peroxidase-containing reagent layer. Otherwise, the sparingly soluble F salt-forming compound can be included in both the peroxidase-containing reagent layer and another layer (or other layers) provided thereover or the spreading layer. If a cation or anion of the sparingly soluble F salt-forming compound disturbs the action of peroxidase or oxidase, said compound can be included in another layer (or other layers) than the layer containing peroxidase or oxidase, and moreover these two or more layers can be separated by provision of an intermediate layer therebetween in a position adjacent to the latter layer.

It will be understood that the necessary amount of the sparingly soluble F salt-forming compound contained in the multilayer analytical element varies depending upon the amount of NaF incorporated into a sample liquid under analysis, as well as upon the amount of the sample liquid applied to the spreading layer of the multilayer analytical element. It will be further understood that the amount of the sparingly soluble F salt-forming compound to be present in the area where the sample liquid is spread and permeates ought to exceed a stoichiometric amount corresponding to a presummed maximum amount of NaF contained in the sample liquid to be introduced into the analytical element. The above-mentioned stoichiometric amount can be roughly determined through calculation. In practice, however, the amount of the sparingly soluble F salt-forming compound contained in the multilayer analytical element generally ranges from 0.1 meq. to 1 eq., preferably from 0.2 meq. to 0.5 eq. based on 1 $m^2$ of the element.

If the analyte is not hydrogen peroxide, but is able to produce hydrogen peroxide by a chemical reaction, an oxidase serving as a catalyst for the reaction between the analyte and oxygen for producing hydrogen peroxide can be included in the reagent layer or another layer. The oxidase included in the multilayer analytical element is necessarily chosen according to nature of the analyte. The oxidase can be employed singly, but a plurality of enzymes (containing at least one oxidase) can be employed for causing a series of continuous reactions. If required, a cofactor or an activator to oxidase and/or its coenzyme can be employed in conjunction with the oxidase.

The oxidase, if required, together with its coenzyme, can be included in a reagent layer containing peroxidase and an indicator or in a layer provided over the reagent layer. Otherwise, it can be included in both the reagent layer containing either of peroxidase and an indicator and one or more layers provided over the reagent layer. Thus, it can be included in two or more layers. The above-mentioned term "a layer provided over the reagent layer" means to include a porous spreading layer.

A reaction of a substrate catalyzed by oxidase requires oxygen. In this respect, since oxygen in a circumferential air is to be introduced from a porous spreading layer to diffuse into other layers, a multilayer analytical element in which an oxidase is included in a layer provided over the reagent layer containing peroxidase and an indicator, particularly in a porous spreading layer or a layer adjacent to the spreading layer so as to accomplish efficient diffusion of oxygen into the layers, as disclosed in GB 2 104 215A, is preferably employed for facilitating efficient progress of the oxidation reaction catalyzed by oxidase. In the case that the analyte is a hydrophobic substance such as a cholesterol ester, the analyte difficulty permeates the layer having a hydrophilic binder polymer. For this reason, oxidase is preferably included inside of a porous spreading layer in such a case.

The oxidase to be contained in a multilayer analytical element of the present invention may be an oxidase utilizing oxygen ($O_2$) as acceptor. Examples of the oxidase utilizing oxygen ($O_2$) as acceptor include the following enzymes: the number included in parenthesis given to the listed enzyme meaning EC number: glycollate oxidase (1.1.3.1), malate oxidase (1.1.3.3), glucose oxidase (1.1.3.4), hexose oxidase (1.1.3.5), cholesterol oxidase (1.1.3.6), aryl-alcohol oxidase (1.1.3.7), L-gulonolactone oxidase (1.1.3.8), galactose oxidase (1.1.3.9), alcohol oxidase (1.1.3.13), L-2-hydroxyacid oxidase (1.1.3.15), aldehyde oxidase (1.2.3.1), xanthine oxidase (1.2.3.2), pyruvate oxidase (1.2.3.3), pyruvate oxidase (CoA acetylating) (1.2.3.6), lathosterol oxidase (1.3.3.2), D-aspartate oxidase (1.4.3.1), L-aminoacid oxidase (1.4.3.2), D-aminoacid oxidase (1.4.3.3), amine oxidase (flavine-containing) (1.4.3.4), pyridoxaminephosphate oxidase (1.4.3.5), amine oxidase (copper-containing) (1.4.3.6), D-glutamate oxidase (1.4.3.7), sarcosine oxidase (1.5.3.1), N-methylaminoacid oxidase (1.5.3.2), $N^6$-methyl-L-lysine oxidase (1.5.3.4), uricase (ureate oxidase) (1.7.3.3), sulfite oxidase (1.8.3.1), ascorbate oxidase (1.10.3.3), 3-hydroxyanthranylate oxidase (1.10.3.5), arginine 2-monooxygenase (1.13.12.1), lysine 2-monooxigenase (1.13.12.2), tryptophan 2-monooxygenase (1.13.12.3), lactate oxidase (lactate 2-monooxygenase) (1.13.12.4), dimethylaniline monooxygenase (1.14.13.8), cholesterol 7α-monooxygenase (1.14.13.17), flavoprotein-linked monooxygenase (1.14.14.1), phenylalanine monooxygenase (1.14.16.1), glycerol oxidase (available from Toyo Jozo Co., Ltd., Japan), and glycine oxidase (described in ENCYCLOPAEDIA CHIMICA, Vol. 3, published in Septemer of 1960 by Kyoritsu Shppan Co., Ltd., Japan).

Examples of a combination of a plurality of enzymes including oxidase include the following:

a combination of glycerol kinase (2.7.1.30) and L-α-glycerophosphate oxidase (1.1.99.5; or disclosed in US Patent 4,166,005, GB 1 585 909, GB 1 590 738, and FR 2 362 396), as disclosed in GB 1 590 738 and FR 2 362 396;

a combination of lipase having triglyceridehydrolyzing activity, glycerol kinase (2.7.1.30), and L-α-glycerophosphate oxidase (1.1.99.5; or disclosed in US Patent 4,166,005, GB 1 585 909, GB 1 590 738, and FR 2 362 396), as disclosed in GB 1 590 738 and FR 2 362 396;

a combination of lipase having esterase activity, protease, and cholesterol oxidase (1.1.3.6), as disclosed in US Patent 3,983,005;

a combination of glutamic-pyruvic transaminase (same as alanine aminotranspherase; 2.6.1.2) and pyruvate oxidase (1.2.3.3), as disclosed in DE 3 222 707 A1; and,

a combination of glutamic-oxaloacetic transaminase (2.6.1.1) (same as glutamine-oxo-acid aminotransferase; 2.6.1.15), oxaloacetate decarboxylase (4.1.1.3), and pyruvate oxidase (1.2.3.3.), as disclosed in DE 3 222 707 A1.

Among these oxidases, preferably employable in the multilayer analytical element of the present invention are glycollate oxidase, malate oxidase, glucose oxidase, hexose oxidase, cholesterol oxidase, L-gulonolactone oxidase, galactose oxidase, xanthine oxidase, pyruvate oxidase, uricase, ascorbate oxidase, lactate oxidase, glycine oxidase, and glycerol oxidase. Also preferred are several combinations including the oxidase exemplified above.

The amount of oxidase to be contained in the multilayer analytical element can be determined principally by a ratio between a presumed maximum amount of a hydrogen peroxide-producible analyte contained in a sample liquid under analysis and activity values of oxidase and peroxidase. Thus, the amount of oxidase can be determined experimentally by those skilled in the art. The amount of oxidase generally ranges from approx. 1,000 $U/m^2$ to approx. 100,000 $U/m^2$, and preferably ranges from approx. 3,000 $U/m^2$ to approx. 50,000 $U/m^2$. In the case that oxidase is glucose oxidase, the amount generally ranges from approx. 2,000 $U/m^2$ to approx. 40,000 $U/m^2$, and preferably ranges from approx. 4,000 $U/m^2$ to approx. 20,000 $U/m^2$.

Each of peroxidase and oxidase has an optiumum pH range respectively where their activities are kept at the maximum value. Their activities are also influenced by an ionic strength and natures of anions and cations present in the vicinity of these enzymes. Accordingly, it is very important for obtaining results satisfactory in the quantitative accuracy, reproducibility, etc. that a multilayer analytical element is so prepared that primarily a pH value of a reagent layer containing peroxidase, a reagent layer containing oxidase, or a reagent layer containing both of peroxidase and oxidase is adjusted to a respective optimum value, and that, if necessary, other factors such as the ionic strength and nature of coexisting ions are adjusted to show optimum conditions. For these reasons, it is especially advantageous that a multilayer analytical element of the present invention contains an acid, an alkali, a salt, a buffer reagent, a dissociating agent, a surface active agent, etc. in one or more of the functional layers such as a porous spreading layer, a reagent layer, and a light-shielding layer. The reagents and their amounts employed for the above-mentioned adjustment of the conditions vary depending upon the analytical purpose and other factors and can be selected in view of the analytical purpose.

7

In order to produce a detectable change in the presence of hydrogen peroxide by incorporating an acid, an alkali or a buffer reagent into the reagent layer containing peroxidase and an indicator, the pH value is preferably adjusted to pH 7.0 of the optimum pH value for peroxidase or a value in the vicinity of pH 7.0, such as, in the range of pH 5.0 (approx.) to pH 9.0 (approx.), preferably pH 6.0 (approx.) to pH 8.0 (approx.).

Different oxidases have their optimum pH values: for instance, pH 5.6 (approx.) for glucose oxidase; pH 6.3 (approx.) for hexose oxidase; pH 5.8 (approx.) for cholesterol oxidase; pH 7.0 (approx.) for galactose oxidase; pH 7.5 (approx.) for alcohol oxidase; pH 7.5—8.5 for uricase; pH 5.6 (approx.) for ascorbate oxidase; and pH 7.5—7.7 for L-α-glycerophosphate oxidase.

The layer containing oxidase is preferably adjusted to have an optimum pH value or a pH value in the vicinity of the optimum value, according to the requirement of nature of oxidase contained therein. Accordingly, an oxidase having an optimum pH value different from an optimum pH value of a peroxidase employed together is preferably included in a layer different from a peroxidase-containing layer. For instance, glucose oxidase can be included in a porous spreading layer of an adhesive layer (described hereinafter). In this case, said layer containing glucose oxidase can be so prepared to show under analytical procedures pH 5.6 of the optimum pH value for glucose oxidase or a value in the vicinity of pH 5.6, such as, in the range of pH 3.6 to pH 7.6, preferably pH 4.0 to pH 7.0, by incorporating appropriately an acid or a buffer thereinto.

Examples of the acid, alkali and buffer reagent employable for the pH adjustment of the reagent layer, other layers or the porous spreading layer include aliphatic hydroxycarboxylic acids (examples: glycolic acid, lactic acid, α-hydroxybutyric acid, and citric, acid, etc.), aliphatic dicarboxylic acids (examples: malonic acid, succinic acid, α-methylglutaric acid, β-methylglutaric acid, 3,3-dimethylglutaric acid, and α,α'-dimethylglutaric acid, etc.), aliphatic monocarboxylic acids (examples: acetic acid, propionic acid, and butyric acid, etc.), lithium hydroxide, sodium hydroxide, potassium hydroxide, buffer reagents described in "Kagakubinran, Kiso-hen (Chemistry Handbook, fundamentals)" edited by the Chemical Society of Japan (Maruzen Tokyo, 1966), pp. 1312—1320 (examples: potassium hydrogen citrate—citric acid, and potassium dihydrogenphosphate potassium hydrogenphosphate, etc.), and buffer reagents described in "Hydrogen Buffers for Biological Research" reported by Norman E. Goods, et al.: Biochemistry, 5(2), 467—477 (1966), "Data for Biochemical Research" Vol. 2 (Oxford at the Clarendon Press, 1966) pp. 476—508, Analytical Biochemistry, 104, 300—310 (1980), etc., (examples: 2-(N-morpholino)ethanesulfonic acid, sodium salt or potassium salt of N-2-hydroxyethylpiperazine-N'-2-hydroxypropane-3-sulfonic acid (Heppso), sodium salt or potassium salt of N - 2 - hydroxyethylpiperazine - N' - 3 - propanesulfonic acid (Epps), etc.), other buffer reagents (examples: sodium or potassium hydrogenmalate, and sodium or potassium monohydrogen - 3,3 - dimethylglutarate, etc.), and inorganic acids (examples: sulfuric acid, and phosphoric acid, etc.). Among these reagents, citric acid, tartaric acid, malic acid, glutaric acid, α-methylglutaric acid, β-methylglutaric acid, α,α'-dimethylglutaric acid and 3,3-dimethylglutaric acid, lithium hydroxide, Heppso Na salt or K salt, and Epps Na salt or K salt are preferred.

The multilayer analytical element of the present invention particularly shows remarkably advantageous feature in that an analyte applied thereto undergoes a photometrically or fluorometrically detectable reaction efficiently in a very short period of time with receiving no disturbance caused by NaF contained in the sample liquid, in the case where a reagent layer containing peroxidase is adjusted to show under analytical procedure a pH value in the vicinity of pH 5.6 (optimum pH value for peroxidase), a reagent layer containing oxidase is adjusted to show under analytical conditions a pH value in the vicinity of the optimum value inherently determined on respective oxidase, and the sparingly soluble F salt-forming compound is included in the peroxidase-containing reagent layer and/or other layers provided thereover.

The porous spreading layer (referred to herein as "spreading layer") of the multilayer analytical element of the present invention is arranged in the outmost position of the element. In other words, the spreading layer is provided in the outmost position far from the support via the reagent layer. The sample liquid is applied or spotted onto the spreading layer. The function of this layer is to supply a sample liquid together with an analyte contained therein into the reagent layer at an approximately constant volume per unit area regardless of its applied volume, that is to say, "metering effect". Thus, this layer acts as a spreader for a sample liquid. Because of such spreading action, a volume of the sample liquid supplied to the reagent layer per unit area is automatically adjusted to a certain value regardless of its applied volume. This means that a sample liquid can be analyzed quantitatively without precise measurement of the volume when applied to the multilayer analytical element. However, it should be understood that the use of the multilayer analytical element of the invention never excludes making precise measurement of a sample liquid in carrying out a quantitative analysis procedure. The precise measurement of a sample liquid is sometimes advantageous to increase accuracy of the analysis.

Example of the porous spreading layer of the present invention include non-fibrous, an isotropically porous layer as disclosed in Japanese Patent Provisional Publication (Jap. Pro. Publn.) No. 49(1974)—53888 (Japanese Patent Publication (Jap. Publn.) No. 53(1978)—21677), US Patent 3,992,158, and GB 1 440 464, for instance, a membrane filter, a blushed polymer layer, and an isotropically porous layer comprising voids defined by fine spheres or particles connected to each other through a binder; an isotropically porous layer having continuous voids and being formed by three-dimensional matrix in which fine spherical beads are bound in point-to-point contact in all directions through binder not swelling with water, as disclosed in US Patent 4,258,001; a fibrous, anisotropically porous spreading layer consisting of water-washed fabrics or hydrophilically processed fabrics, as disclosed in US Patent 4,292,272; a fibrous, anisotropically porous spreading layer consisting of fabrics having physically activated surfaces, as disclosed in GB 2 087 074A; and a fibrous, anisotropic porous spreading layer consisting of paper, paper filter or non-woven fabrics containing synthetic polymer fiber pulps, as disclosed in GB 2 087 074A. Any of these spreading layers can be provided to the analytical element of the invention in manners disclosed in these patent specifications. Otherwise, a membrane filter or blushed polymer layer containing titanium dioxide, zinc oxide, or barium sulfate in the form of fine powder disclosed in US Patent 3,992,158 and GB 1 440 464 can be employed in the analytical element of the invention for serving as a spreading layer and also as a light-shielding layer (radiation-blocking layer, white background layer, or light-reflecting layer), details being given hereinafter. Further, a membrane filter or a blushed polymer layer containing carbon black can be employed as a spreading layer which also serves as a light-shielding layer, as described hereinafter. If a sample liquid is a whole blood, the spreading layer preferably is the aforementioned isotropically porous layer having continuous voids and being formed by three-dimensional matrix, or the fibrous, anisotropically porous spreading layer.

The multilayer analytical element of the invention can be provided with a light-shielding layer (radiation-blocking layer, background layer, or light-reflecting layer) capable of allowing permeation of water and analyte, between the reagent layer and the spreading layer.

The light-shielding layer is advantageously provided if the analytical element is employed for analysis of a sample liquid containing colored particles such as the whole blood containing red corpuscles. In more detail, colored particles positioned on one side of the light-shielding layer are photometrically shielded by the light-shielding layer from observation through the transparent support. Accordingly, the colorimetric or fluorometric measurement is not interferred with by the presence of colored particles. The light-shielding layer can be composed of a fine powder such as finely particulated titanium dioxide, barium sulfate, zinc oxide, aluminum or carbon black dispersed within a water- and analyte-permeable, hydrophilic polymer binder. The light-shielding layer has a thickness in the range of from 5 to 100 μm, preferably 5 to 30 μm, and allows a liquid component and an analyte of a sample solution passing therethrough. The polymer binder for the preparation of the light-shielding layer can be optionally selected from the hydrophilic polymers described hereinbefore in connection with the binder for the reagent layer.

The light-shielding layer can be a porous light-shielding layer consisting of membrane filter (blushed polymer layer) containing light-shielding particles such as finely particulated titanium dioxide, zinc oxide, barium sulfate and carbon black. The porous light-shielding layer can be provided to the analytical element in the manner disclosed in US Patents 3,992,158 and 4,166,093, and GB 1 440 464.

The multilayer analytical element of the present invention can be provided with an adhesive layer for superposing the spreading layer on the reagent layer, the light-shielding layer, or other optionally-placed layers under increased adhesion to form an integrally laminated structure. The adhesive layer is preferably provided if the spreading layer is made of a porous sheet, a porous film or a porous membrane.

The adhesive layer can be produced from one or more of the water- and analyte-permeable hydrophilic polymer described hereinbefore in connection with the binder for the reagent layer. The adhesion of the spreading layer to the adhesive layer can be carried out by placing a porous sheet, film or membrane under pressure on an adhesive layer consisting of half-dried hydrophilic polymer or wetted with water or an aqueous solution containing a surface active agent. The adhesive layer has a thickness in the range of from 0.5—15 μm, preferably 0.5 to 5 μm.

The multilayer analytical element of the invention can be provided, if desired, with a variety of layers such as a barrier layer or a liquid-blocking layer disclosed in US Patent (Re) 30,267, Japanese Patent Provisional Publication No. 58(1983)—77660 and GB 2 114 737A, etc.; a detection layer or a mordant layer disclosed in US Patents 4,042,335 and 4,144,306, etc.; a migration inhibiting layer disclosed in US Patent 4,166,093; the intermediate layer disclosed in US Patents 4,098,574 and 4,042,335, etc.; a protein-permeable hydrophilic binder polymer layer disclosed in US Patents 4,144,306 and 4,268,563, GB 1 474 285, EP 0044775 A1, US Patent 4,333,733, Japanese Patent Application No. 57(1982)—61936, etc.; a reagent layer comprising hydrophobic particles in which a reagent is contained under dispersion in a hydrophilic binder disclosed in US Patent 4,356,149; a porous material (patch) for application and supply of a sample liquid in a certain limited area disclosed in DE 3 133 538 A1; and a porous layer having a certain limited area and containing a reagent such as enzyme disclosed in DE 3 222 707 A1.

The multilayer analytical element of the present invention comprises, in sequence, a spreading layer, a reagent layer containing peroxidase, and a transparent support.

Among arrangements of the various layers, preferred are:

an arrangement comprising, in sequence, a spreading layer, a light-shielding layer, a reagent layer containing peroxidase, and a transparent support;

an arrangement comprising, in sequence, a spreading layer, an adhesive layer, a light-shielding layer, a reagent layer containing peroxidase, and a transparent support;

an arrangement comprising, in sequence, a spreading layer, a light-shielding layer containing oxidase, a reagent layer containing peroxidase, and a transparent support;

an arrangement comprising, in sequence, a spreading layer, an adhesive layer containing oxidase, a light-shielding layer, a reagent layer containing peroxidase, and a transparent support;

an arrangement comprising, in sequence, a spreading layer containing oxidase, an adhesive layer containing oxidase, a light-shielding layer, a reagent layer containing peroxidase, and a transparent support;

an arrangement comprising, in sequence, a spreading layer, an adhesive layer containing oxidase, a light-shielding layer containing oxidase, a reagent layer containing peroxidase, and a transparent support;

an arrangement comprising, in sequence, a spreading layer, a light-shielding layer, a reagent layer containing both of peroxidase and oxidase, and a transparent support;

an arrangement comprising, in sequence, a spreading layer, an adhesive layer, a light-shielding layer containing oxidase, a reagent layer containing both of peroxidase and oxidase, and a transparent support;

an arrangement comprising, in sequence, a spreading layer, a reagent layer containing oxidase, a light-shielding layer, a reagent layer containing both of peroxidase and oxidase, and a transparent support; and

other variations of the above-described arrangements.

The multilayer analytical element of the present invention can be prepared in the manners disclosed in the aforementioned patent specifications. Examples of the detailed procedures are described in the hereinafter-given working examples.

The multilayer analytical element of the invention can be employed in quantitative analysis of analytes contained in a variety of sample liquids in the same manners as disclosed in the aforementioned patent specifications. The analytical element of the invention is preferably received in a slide frame and employed in the form of an analytical slide as disclosed in Japanese Utility Model Provisional Publication No. 54(1979)—162294, US Patents 4,387,990 and 4,169,751, and Japanese Patent Provisional Publication No. 57(1982)—63452. Otherwise, the analytical element of the invention is preferably provided with a sample liquid-spreading assistant material (or a sample-spreading assisting element) on the porous spreading layer to form an analytical slide as disclosed in Japanese Patent Provisional Publication No. 57(1982)—182648. The analytical element in the form of such a slide is preferred in all aspects, namely, preparation, transportation, storage, measurement procedure and so forth.

The present invention will be further described by the following examples, which are not given to restrict the invention.

Example 1

A reagent layer for quantitative analysis of glucose concentration in blood, having a thickness of 15 µm (dry basis), was formed on a transparent polyethylene terephthalate (PET) film (thickness: 185 µm) having a gelatin subbing layer, by coating the following composition thereon.

| | |
|---|---|
| Peroxidase | 25000 IU |
| 1,7-Dihydroxynaphthalene | 5 g. |
| 4-Aminoantipyrine | 5 g. |
| Gelatin | 200 g. |
| Nonion HS 210 (polyoxyethylenenonylphenyl ether; produced by Nippon Oils & Fats Co., Ltd., Japan) | 2 g. |

On the reagent layer, a light-shielding layer having a thickness of 15 µm (dry basis) was formed by coating thereon a mixture of 8 g. of powdery titanium dioxide containing 0.2 g. of Nonion HS 210 and 50,000 IU of glucose oxidase dispersed in 1 g. of gelatin.

On the light-shielding layer, an adhesive layer having a thickness of 4 µm (dry basis) was formed by coating thereon a coating mixture of 4 g. of gelatin, 2 g. of calcium acetate, and 0.2 g. of Nonion HS 210 in 100 ml. of water.

The adhesive layer was wetted with water in an amount of 30 g./m$^2$, and subsequently a cotton broadcloth (100% cotton, woven from cotton yarn of 100 count, manufactured by Toyobo Co., Ltd., Japan) was pressed onto the adhesive layer and dried to give a porous spreading layer. Thus, a multilayer analytical element for quantitative analysis of glucose was prepared.

The analytical element was cut to obtain a square tip (1.5 cm×1.5 cm), which was in turn inserted into a plastic mount, to prepare an analytical slide for quantitative analysis of glucose.

The analytical slide was examined for resistance to the interferring disturbance of NaF in the following manner.

The sample liquid for analysis was prepared by adding different amounts of NaF to a human plasma containing 236 mg./dl. of glucose which was obtained by collecting a blood in the presence of heparin and then subjecting the blood to centrifugal separation. The sample liquid in an amount of 10 µl. was spotted on the spreading layer of the analytical slide, which was then incubated at 37°C for 6 min., and subjected to reflective spectroscopy at 500 nm. The results are set forth in Table 1, in which the optical density value of color formation is calculated by subtracting a fog optical density value (an optical density value of color formation observed in a measurement of an incubated (37°C for 6 min.) analytical slide on which 7 wt.% of

10

aqueous human albumin solution (containing no glucose and no NaF) was spotted), from an optical density value observed on the sample liquid (human plasma) under analysis.

The results in Table 1 clearly indicate that the analytical element prepared as above gave almost same optical density value (color formation) in the presence of NaF in amounts ranging from 0 to 10 mg./dl.

Comparison Example 1

A control analytical slide was prepared in the same manner as in Example 1 except for incorporating no calcium acetate into the adhesive layer.

On the spreading layer of the so prepared analytical slide were spotted, in the same manner as in Example 1, the same human plasmas of different NaF concentrations. The analytical slide was then incubated and subjected to reflective spectroscopy in the same manner as in Example 1. The results are set forth in Table 1.

The results indicate that the optical density value obtained in the use of the analytical element prepared as above was relatively high, as far as the NaF concentration was at a low level, and the optical density value decreased as the amount of NaF increased.

TABLE 1

| Analytical Element | Amount of Added NaF (mg./ml.) | | | | |
|---|---|---|---|---|---|
| | 0 | 2.0 | 4.0 | 8.0 | 10.0 |
| Example 1 (According to the invention) | 1.02 | 1.04 | 1.04 | 1.02 | 1.06 |
| Comparison Example 1 (Control) | 0.99 | 0.92 | 0.86 | 0.85 | 0.83 |

Remark: The value is indicated by optical density value of color formation.

Example 2

An analytical slide for quantitative analysis of glucose was prepared in the same manner as in Example 1 except for replacing calcium acetate with 2 g. of calcium chloride (accordingly, the amount of $Ca^{2+}$ ion was approx. 1.4 times as much as that in the case of Example 1).

On the spreading layer of the so prepared analytical slide were spotted in the same manner as in Example 1, the same human plasmas of different NaF concentrations. The analytical slide was then incubated and subjected to reflective spectroscopy in the same manner as in Example 1. The results are set forth in Table 2.

The results in Table 2 clearly indicate that the analytical element prepared as above was satisfactorily resistant to the interferring disturbance of NaF.

Comparison Example 2

A control analytical slide was prepared in the same manner as in Example 2 except for incorporating no calcium chloride into the adhesive layer.

On the spreading layer of the so prepared analytical slide were spotted in the same manner as in Example 1, the same human plasmas of different NaF concentrations. The analytical slide was then incubated and subjected to reflective spectroscopy in the same manner as in Example 1. The results are set forth in Table 2.

The results indicate that the optical density value obtained in the use of the analytical element prepared as above was extremely influenced by the interferring disturbance of NaF in the same manner as in Comparison Example 1.

# 0 101 945

TABLE 2

| Analytical Element | Amount of Added NaF (mg./ml.) | | | | | |
|---|---|---|---|---|---|---|
| | 0 | 2.0 | 4.0 | 8.0 | 10.0 | 14.0 |
| Example 2 (According to the invention) | 234 | 233 | 238 | 241 | 238 | 232 |
| Comparison Example 2 (Control) | 226 | 238 | 244 | 231 | 225 | 198 |

Remark: The value is indicated by an amount of glucose (mg./dl.) determined by the analysis of the blood plasma containing 236 mg./dl (this value was separately determined by glucose oxidase—oxygen electrode method).

Example 3

An analytical slide for quantitative analysis of glucose was prepared in the same manner as in Example 1 except for replacing calcium acetate with 2 g. of barium chloride (accordingly, the amount of $Ba^{2+}$ ion per the unit area was approx. 0.9 time (equivalency basis) as much as the amount of $Ca^{2+}$ employed in the case of Example 1).

On the spreading layer of the so prepared analytical slide were spotted in the same manner as in Example 1, the same human plasmas of different NaF concentrations. The analytical slide was then incubated and subjected to reflective spectroscopy in the same manner as in Example 1. The results indicated that the analytical element prepared as above was satisfactory resistant to the interferring disturbance of NaF in the same manner as in Example 1.

Example 4

An analytical slide for quantitative analysis of glucose was prepared in the same manner as in Example 1 except for incorporating calcium acetate into the light-shielding layer in the amount of 1.5 g./m² in place of into the adhesive layer.

On the spreading layer of the so prepared analytical slide were spotted in the same manner as in Example 1, the same human plasmas of different NaF concentrations. The analytical slide was then incubated and subjected to reflective spectroscopy in the same manner as in Example 1. The results indicated that the analytical element prepared as above was satisfactorily resistant to the interferring disturbance of NaF in the same manner as in Example 1.

Example 5

An analytical slide for quantitative analysis of glucose was prepared in the same manner as in Example 1 except for employing an aqueous solution containing 10% calcium acetate as the aqueous wetting liquid for wetting the adhesive layer for superposing the broadcloth thereon in place of incorporating calcium acetate in the adhesive layer.

On the spreading layer of the so prepared analytical slide were spotted, in the same manner as in Example 1, the same human plasmas of different NaF concentrations. The analytical slide was then incubated and subjected to reflective spectroscopy in the same manner as in Example 1. The results indicated that the analytical element prepared as above was satisfactorily resistant to the interferring disturbance of NaF, in the same manner as in Example 1.

Example 6

An analytical slide for quantitative analysis of cholesterol was prepared in the same manner as in Example 1 except for replacing glucose oxidase (incorporated into the adhesive layer) with the same unit amount of cholesterol oxidase which was incorporated into the light-shielding layer.

On the spreading layer of the so prepared analytical slide were spotted in the same manner as in Example 1, the same human plasmas of different NaF concentrations. The analytical slide was then incubated and subjected to reflective spectroscopy in the same manner as in Example 1.

Also prepared was a control analytical slide containing no calcium acetate, and this slide was then evaluated in the same manner.

The results indicated that the analytical element containing calcium acetate was satisfactorily resistant to the interferring disturbance of NaF, while the control analytical element containing no calcium acetate was noticeably disturbed by the presence of NaF.

12

Example 7

The pH values under analytical conditions (reaction conditions) were measured on the slides prepared in Example 1 and Comparison Example 1 through the experiments described below, in which the pH measurements was carried out by means of a pH meter HM 15 (manufactured by Toa Denpa Co., Ltd., Japan) connected with an electrode for surface pH measurement GS-165F.

On the analytical slide for quantitative analysis of glucose was spotted 10 μl. of a sample liquid such as distilled water, a fresh control human plasma, etc. Upon the sample liquid was spread in the element, the porous spreading layer was removed from the element. Subsequently, the pH electrode was brought into contact with the free surface of the adhesive layer of the element, and pH values were continuously measured for a period from 1 min. (calculated starting from the time of removal of the spreading layer) to 9 min., by every one minute. The electrode was calibrated by placing it in close contact with 10 μl. of standard aqueous phosphate solutions of pH 4.01 and 6.86 (25°C) spotted on the PET film. The results of pH measurements obtained in the cases of using a fresh human plasma (A) and distilled water (B) as the sample liquids are graphically illustrated in Fig. 1.

The results illustrated in Fig. 1 clearly indicate that the pH value reached an equilibrium value within 2—3 min. for respective case.

Example 8

An analytical slide for quantitative analysis of glucose was prepared in the same manner as in Example 1 except for varying the pH value by adding different amounts of 3,3-dimethylglutaric acid in the presence of 1.0 g./m² of calcium acetate.

The so prepared analytical slide was subjected to the pH measurement on the adhesive layer in the same manner as in Example 7. The sample liquid was the same human plasma as that employed in Example 7 and spotted in the amount of 10 μl.

The sample liquids containing NaF at the concentration of 0 (none), 1.0 and 3 mg./ml. were prepared by introducing a human plasma obtained by collecting a blood in the presence of heparin and then subjecting the blood to centrifugal separation, into test tubes containing different amounts of NaF. The sample liquid in the amount of 110 μl. was spotted on the porous speading layer of the analytical slide, which was then incubated at 37°C for 6 min., and subjected to reflective spectroscopy at 500 nm. The results are set forth in Table 3.

The results set forth in Table 3 clearly indicate that the analytical element prepared as above was almost free from the interferring disturbance by NaF independent of pH value.

TABLE 3

| Amount of 3,3-dimethylgluta-ric acid in adhesive layer | pH value of adhesive layer (observed) | Amount of NaF in Human Plasma (mg./ml.) | | |
|---|---|---|---|---|
| | | 0 | 1.0 | 3.0 |
| 0 | 6.3 | 0.77 | 0.78 | 0.78 |
| 0.02 g./m² | 5.9 | 0.77 | 0.78 | 0.77 |
| 0.04 g./m² | 5.8 | 0.77 | 0.77 | 0.78 |
| 0.08 g./m² | 5.4 | 0.76 | 0.78 | 0.76 |
| 0.12 g./m² | 5.2 | 0.75 | 0.75 | 0.76 |

**Claims**

1. A multilayer analytical element comprising a water-impermeable, light-transmissive support, a reagent layer which comprises a hydrophilic binder polymer and a color-forming indicator composition for detection of hydrogen peroxide, this reagent layer containing at least peroxidase, and a porous spreading layer, which are superposed in this order,

in which at least one layer other than said support contains 0.1 meq./m² to 1 eq./m² of a compound comprising a cation capable of forming in combination with $F^-$ ion a sparingly water-soluble salt having solubility of not more than 0.2 g. in 100 g. of water at 25°C.

2. The multilayer analytical element as claimed in Claim 1, in which said cation is at least one cation selected from $Mg^{2+}$, $Ca^{2+}$, $Sr^{2+}$, $Ba^{2+}$, $Pb^{2+}$, $Fe^{2+}$, $Cu^{2+}$, $Bi^{3+}$, $Fe^{3+}$, $V^{3+}$, $Na_3Al^{6+}$, $K_2Si^{6+}$, $CaSi^{6+}$, and $BaSi^{6+}$.

3. The multilayer analytical element as claimed in Claim 1 or 2, in which said reagent layer contains peroxidase and 4-aminoantipyrine or a derivative thereof.

4. The multilayer analytical element as claimed in any one of Claims 1 to 3, in which oxidase is

13

**0 101 945**

contained in said reagent layer or in a layer in which oxidase is contained in said reagent layer or in a layer provided over the reagent layer or in both the reagent layer containing either of peroxidase and an indicator and one or more layers provided over the reagent layer.

5. The multilayer analytical element as claimed in Claim 4, in which said oxidase is selected from glycollate oxidase, malate oxidase, glucose oxidase, hexose oxidase, cholesterol oxidase, L-gulonolactone oxidase, galactose oxidase, xanthine oxidase, pyruvate oxidase, uricase, ascorbate oxidase, lactate oxidase, glycine oxidase, and glycerol oxidase.

6. The multilayer analytical element as claimed in Claim 5, in which said oxidase is glucose oxidase.

7. The multilayer analytical element is claimed in Claim 5, in which said oxidase is cholesterol oxidase.

**Patentansprüche**

1. Mehrschichtiges analytisches Element mit einem wasserundurchlässigen, lichtdurchlässigen Träger, einer Reagenzschicht enthaltend ein hydrophiles Bindemittel-Polymeres und eine farbbildende Indikatorzusammensetzung zum Nachweis von Hydroperoxid, wobei diese Reagenzschicht mindestens Peroxidase enthält und mit einer porösen Ausbreitungsschicht, welche in dieser Reihenfolge aufeinandergelegt sind, wobei mindestens eine andere Schicht als der genannte Träger 0,1 mÄq./m² bis 1 Äq./m² einer Verbindung enthält, die ein Kation besitzt, welches dazu imstande ist, in Kombination mit einem F⁻-Ion ein kaum wasserlösliches Salz zu bilden, das eine Löslichkeit von nicht mehr als 0,2 g in 100 g Wasser bei 25°C besitzt.

2. Mehrschichtiges analytisches Element nach Anspruch 1, dadurch gekennzeichnet, daß das genannte Kation mindestens ein Kation ausgewählt aus $Mg^{2+}$, $Ca^{2+}$, $Sr^{2+}$, $Ba^{2+}$, $Pb^{2+}$, $Fe^{2+}$, $Cu^{2+}$, $Bi^{3+}$, $Fe^{3+}$, $V^{3+}$, $Na_3Al^{6+}$, $K_2Si^{6+}$, $CaSi^{6+}$ und $BaSi^{6+}$ ist.

3. Mehrschichtiges analytisches Element nach Anspruch 1 oder 2, dadurch gekennzeichnet, daß die genannte Reagenzschicht Peroxidase und 4-Aminoantipyrin oder ein Derivat davon enthält.

4. Mehrschichtiges analytisches Element nach einem der Ansprüche 1 bis 3, dadurch gekennzeichnet, daß die Oxidase in der genannten Reagenzschicht oder in einer Schicht, in der Oxidase in der genannten Reagenzschicht enthalten ist, oder in einer Schicht, die über der Reagenzschicht vorgesehen ist, oder in sowohl der Reagenzschicht, die entweder Peroxidase oder einen Indikator enthält, und einer oder mehreren Schichten, die über der Reagenzschicht vorgesehen sind, enthalten ist.

5. Mehrschichtiges analytisches Element nach Anspruch 4, dadurch gekennzeichnet, daß die genannte Oxidase aus Glycollatoxidase, Malatoxidase, Glucoseoxidase, Hexoseoxidase, Cholesterinoxidase, L-Gulonolactonoxidase, Galactoseoxidase, Xanthinoxidase, Pyruvatoxidase, Uricase, Ascorbatoxidase, Lactatoxidase, Glycinoxidase und Glycerinoxidase ausgewählt ist.

6. Mehrschichtiges analytisches Element nach Anspruch 5, dadurch gekennzeichnet, daß die genannte Oxidase Glucoseoxidase ist.

7. Mehrschichtiges analytisches Element nach Anspruch 5, dadurch gekennzeichnet, daß die genannte Oxidase Cholesterinoxidase ist.

**Revendications**

1. Elément analytique à plusieurs couches comprenant un support transmettant la lumière et imperméable à l'eau, une couche de réactif qui comprend un polymère liant hydrophile et une composition indicatrice formant une couleur pour la détection du peroxyde d'hydrogène, cette couche de réactif contenant au moins une peroxydase et une couche d'étendage poreuse, qui sont superposées dans cet ordre, dans lequel au moins une couche autre que celle du support contient 0,1 meq./m² à 1 eq/m² d'un composé comprenant un cation capable de former en association avec l'ion F⁻ un sel très pur soluble dans l'eau ayant une solubilité qui ne dépasse pas 0,2 g dans 100 g d'eau à 25°C.

2. Elément analytique à plusieurs couches suivant la revendication 1, caractérisé en ce que ce cation est au moins un cation choisi parmi $Mg^{2+}$, $Ca^{2+}$, $Sr^{2+}$, $Ba^{2+}$, $Pb^{2+}$, $Fe^{2+}$, $Cu^{2+}$, $Bi^{3+}$, $Fe^{3+}$, $V^{3+}$, $Na_3Al^{6+}$, $K_2Si^{6+}$, $CaSi^{6+}$ et $BaSi^{6+}$.

3. Elément analytique à plusieurs couches suivant la revendication 1 ou 2, caractérisé en ce que cette couche de réactif contient une peroxydase et de la 4-aminoantipyrine ou un dérivé de celle-ci.

4. Elément analytique à plusieurs couches, suivant l'une quelconque des revendications 1 à 3, caractérisé en ce que l'oxydase est contenue dans la couche de réactif ou dans une couche disposée sur la couche de réactif, ou bien à la fois dans la couche de réactif contenant une quelconque peroxydase et un indicateur et dans une ou plusieurs couches disposées sur la couche de réactif.

5. Elément analytique à plusieurs couches suivant la revendication 4, caractérisé en ce que l'oxydase est choisie parmi la glycollate oxydase, la malate oxydase, la glucose oxydase, l'hexose oxydase, le cholestérol oxydase, la L-gulonolactone oxydase, la galactose oxydase, la xanthine oxydase, la pyruvate oxydase, l'uricase, l'ascorbate oxydase, la lactate oxydase, la glycine oxydase, et le glycérol oxydase.

6. Elément analytique à plusieurs couches suivant la revendication 5, caractérisé en ce que l'oxydase est la glucose oxydase.

7. Elément analytique à plusieurs couches suivant la revendication 5, caractérisé en ce que l'oxydase est le cholestérol oxydase.

14

# FIG.1